(19) Europäisches Patentamt
European Patent Office
Office européen des brevets

(11) Publication number: **0 191 166**
**B1**

(12) **EUROPEAN PATENT SPECIFICATION**

(45) Date of publication of patent specification: **06.02.91**

(21) Application number: **85115383.3**

(22) Date of filing: **04.12.85**

(51) Int. Cl.[5]: **C 07 D 311/92,**
C 07 D 493/04,
C 07 D 497/04,
C 07 D 405/04, A 61 K 31/35,
A 61 K 31/38, A 61 K 31/445

(54) **Aminoacyllabdanes, a process and intermediates for their preparation and their use as medicaments.**

(30) Priority: **14.12.84 US 681779**

(43) Date of publication of application:
**20.08.86 Bulletin 86/34**

(45) Publication of the grant of the patent:
**06.02.91 Bulletin 91/06**

(84) Designated Contracting States:
**AT BE CH DE FR GB IT LI LU NL SE**

(56) References cited:
**DE-A-2 654 796**

(73) Proprietor: **HOECHST-ROUSSEL PHARMACEUTICALS INCORPORATED**
**Route 202-206 North**
**Somerville New Jersey 08876 (US)**

(72) Inventor: **Kosley, Raymond W., Jr.**
**659 Cedarbrook Road**
**Bridgewater New Jersey 08807 (US)**
Inventor: **Cherill, Robert J.**
**80 Kossuth Street**
**Somerset New Jersey 08873 (US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to aminoacyllabdanes of formula *1*

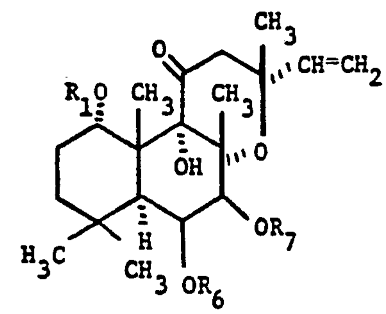

wherein $R_1$ is $R_2R_3NCHR_4CO$ wherein $R_2$ is hydrogen, $C_1$—$C_6$-alkyl or benzyl, $R_3$ is hydrogen or $C_1$—$C_6$-alkyl and $R_4$ is hydrogen, $C_1$—$C_6$-alkyl or benzyl or $R_2$ and $R_3$ taken together with the nitrogen atom to which they are attached form a group of the formula

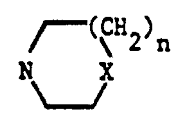

wherein X is CO, O, S, SO, $SO_2$; a group of the formula $CHR_9$ wherein $R_9$ is hydrogen, $C_1$—$C_6$-alkyl or a group of the formula $OR_{10}$ wherein $R_{10}$ is hydrogen or $COR_{11}$ wherein $R_{11}$ is $C_1$—$C_6$-alkyl; or a group of the formula $NR_{12}$ wherein the $R_{12}$ is $C_1$—$C_6$-alkyl; n is 0 or 1; $R_6$ is hydrogen or a group of the formula $R_5CO$ wherein $R_5$ is hydrogen or $C_1$—$C_6$-alkyl; $R_7$ is hydrogen or a group of the formula $R_8CO$ wherein $R_8$ is hydrogen or $C_1$—$C_6$-alkyl or $R_6$ and $R_7$ taken together form a group of the formula CO or SO; the optical and geometric isomers thereof, or a pharmaceutically acceptable acid addition salt thereof, which are useful for reducing intraocular pressure, alone or in combination with inert adjuvants.

The present invention relates particularly to compounds of the formula *1*, wherein $R_2$ and $R_3$ are both $C_1$—$C_6$-alkyl, or $R_2$ and $R_3$ taken together with the nitrogen atom to which they are attached form a group of the formula

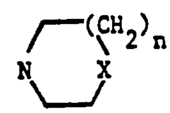

wherein X is O, S, a group of the formula $CHR_9$ where $R_9$ is hydrogen or a group of the formula $OR_{10}$ where $R_{10}$ is hydrogen and n is 0 or 1, $R_4$ is hydrogen or $C_1$—$C_6$-alkyl, $R_6$ is hydrogen and $R_7$ is a group of the formula $R_8CO$ where $R_8$ is hydrogen or $C_1$—$C_6$-alkyl.

The present invention also relates to compounds of formula

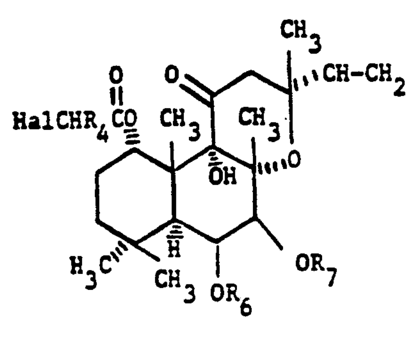

2

wherein $R_4$ is hydrogen, lower alkyl or benzyl; $R_6$ is hydrogen or a group of the formula $R_5CO$ wherein $R_5$ is hydrogen or $C_1$—$C_6$-alkyl $R_7$ is hydrogen or a group of the formula $R_8CO$ wherein $R_8$ is hydrogen or $C_1$—$C_6$-alkyl Hal is chloro or bromo; or the optical and geometric isomers thereof, which are useful as intermediates for the preparation of the aminoacyllabdanes of the present invention.

A compound of formula *2* wherein Hal is bromo is preferred. As used through the specification and appended claims, the term "alkyl" refers to a straight chain hydrocarbon radical containing no unsaturation and having 1 to 8 carbon atoms such as methyl, ethyl, 1-propyl, 2-propyl, 2-methylpropyl, 1-pentyl, 2-pentyl, 3-hexyl, 4-heptyl, 2-octyl; the term "alkanol" refers to a compound formed by a combination of an alkyl group and a hydroxy radical. Examples of alkanols are methanol, ethanol, 1- and 2-propanol, 1,2-dimethylethanol, hexanol, octanol.

The term "alkanolic acid" refers to a compound formed by combination of a carboxyl group with a hydrogen atom or alkyl group. Examples of alkanoic acids are formic acid, acetic acid, propanoic acid, 2,2-dimethylacetic acid, octanoic acid; the term "halogen" refers to a member of the family consisting of fluorine, chlorine, bromine or iodine. The term "alkanoyl" refers to the radical formed by removal of the hydroxyl function from an alkanoic acid. Examples of alkanoyl groups are formyl, acetyl, propionyl, 2,2-dimethylacetyl, hexanoyl, octanoyl.

In the formula presented herein the various substituents are illustrated as joined to the labdane nucleus by one of two notations: a solid line (—) indicating a substituent which is in the β-orientation (i.e., above the plane of the molecule) and a broken line (- - -) indicating a substituent which is in the α-orientation (i.e., below the plane of the molecule). The formulas have all been drawn to show the compounds in their absolute sterochemical configuration. Inasmuch as the starting materials having a labdane nucleus are naturally occurring or are derived from naturally occurring materials, they, as well as the final products, have a labdane nucleus existing in the single absolute configuration depicted herein. The processes of the present invention, however, are intended to apply as well to the synthesis of labdanes of the racemic series.

In addition to the optical centers of the labdane nucleus, the substituents thereon may also contain chiral centers contributing to the optical properties of the compounds of the present invention and providing a means for the resolution thereof by conventional methods, for example, by the use of optically active acids. The present invention comprehends all optical isomers and racemic forms of the compounds of the present invention, where such compounds have chiral centers in addition to those of the labdane nucleus.

The novel aminoacyllabdanes of the present invention are synthesized by the processes illustrated in the Reaction Scheme.

To prepare an aminoacyllabdane *4* wherein $R_7$ is $C_1$—$C_7$-alkanoyl, a 1-hydroxyacyllabdane *3* wherein $R_7$ is $C_1$—$C_7$-alkanoyl is acylated with a haloalkylcarbonyl halide of formula *8*

$$HalCHR_4COHal \qquad 8$$

wherein $R_4$ and Hal are as hereinbefore described to provide a 1-haloalkanoyloxyabdane *2* which is condensed with an amine of formula *9*

$$R_2R_3NH \qquad 9$$

to afford *4*.

The acylation of hydroxylabdane *3* is readily accomplished by treating a hydroxylabdane *3* with a halo-$C_1$—$C_6$-alkylcarbonyl halide *8* such as a bromo-$C_1$—$C_6$-alkylcarbonyl bromide or a chloro-$C_1$—$C_6$-alkylcarbonyl chloride, a bromo-$C_1$—$C_6$-alkylcarbonyl bromide being preferred, in a halohydrocarbon in the presence of a tertiary amine. Among halocarbons, there may be mentioned dichloromethane, trichloromethane, 1,1- and 1,2-dichloromethane and 1,1- and 1,2-dichloroethane. Dichloromethane is the preferred halohydrocarbon. Among tertiary amines, there may be mentioned, for example, 4-dimethylaminopyridine and N,N-dimethylaniline. N,N-Dimethylaniline is the preferred amine. While the temperature at which the acylation is performed is not narrowly critical, it is preferred to conduct the reaction at a temperature within the range of about −0° to about 50°C. It is most preferred to perform the acylation at a temperature within the range of about 0° to 25°C.

The condensation is effected by treating a halo-$C_1$—$C_6$-alkanoyloxyabdane *2* with a primary or secondary amine *9* in an $C_1$—$C_6$-alkyl alkanoate or halohydrocarbon, or a mixture thereof. Included among $C_1$—$C_6$-alkyl alkanoates are methyl acetate, ethyl acetate and ethyl propanoate. Included among halohydrocarbons are dichloromethane, trichloromethane, 1,1- and 1,2-dichlormethane. Ethyl acetate and dichloromethane are the preferred solvents. The condensation is preferably performed in the absence of added base. An alkali metal bicarbonate such as lithium, sodium or potassium bicarbonate may, however, be utilized. The condensation temperature is not critical. The conversion proceeds readily at a temperature within the range of about 0° to about 50°C. A reaction temperature of about 25°C is preferred.

To provide an aminoacyllabdane *5* wherein $R_6$ is $C_1$—$C_7$-alkanoyl, aminoacyllabdane *4* may be rearranged, for example, by treating *4* with lithium 1,1,1,3,3,3-hexamethyldisilazide in an etheral solvent such as tetrahydrofuran at a temperature of about 0°C.

To provide an aminoacyllabdane *6* having free 6β,7β-hydroxy groups, one may treat 1α,6β,7β,9α-tetrahydroxylabdane *3* wherein $R_7$ is hydrogen with halo-$C_1$—$C_6$-alkylcarbonyl halide *8* under reaction conditions substantially similar to those employed for the conversion of trihydroxylabdane *3* wherein $R_7$ is $C_1$—$C_7$-alkanoyl to aminoacylabdane *4* wherein $R_7$ is $C_1$—$C_7$-alkanoyl.

To synthesize an aminoacyllabdane *8* wherein $R_6$ and $R_7$ are $C_1$—$C_7$-alkanoyl, one may acylate an aminoacyllabdane *5* with an alkanoic acid of formula *10*

$$R_5(R_8)CO_2H \qquad 10$$

wherein $R_5$ and $R_8$ are as hereinbefore described, an anhydride thereof of formula *11*

$$R_5(R_8)\overset{\displaystyle O}{\overset{\|}{C}}-O-\overset{\displaystyle O}{\overset{\|}{C}}-(R_8)R_5 \qquad 11$$

wherein $R_5$ and $R_8$ are as hereinbefore described, or mixtures thereof, or the halide thereof of formula *12*

$$R_5(R_8)COHal \qquad 12$$

wherein $R_5$, $R_8$ and Hal are as hereinbefore described. The acylation may be performed in the presence of a basic catalyst such as, for example, pyridine, lutidine or collidine at a reduced temperature within the range of about 0° to 25°C.

In the event a carboxylic acid of formula *10* is employed in the acylation step, a carbodiimide such as, for example, dicyclohexylcarbodiimide, may be utilized.

Aminoacyllabdanes of formula *7* wherein Y is CO or SO may be prepared by treating a dihydroxyl-abdane *6* with a compound of formula *13*

$$Hal—Y—Hal \qquad 13$$

wherein Y is CO or SO and Hal is as hereinbefore described in the presence of an aromatic amine such as pyridine, lutidine or s-collidine at a reduced temperature of about −10 to about 10°C.

The labdane starting materials for the processes of the present invention, i.e., labdanes of formula *3* wherein $R_7$ is hydrogen or a group of the formula $R_8CO$ wherein $R_8$ is hydrogen or $C_1$—$C_6$-alkyl, are described in U.S. Patent 4,134,986, issued January 16, 1979 to B. S. Bajwa, et al.

The aminoacyllabdanes of the present invention are useful in the treatment of elevated intraocular pressure by virtue of their ability to reduce intraocular pressure as determined by the method described by J. Caprioli, et al., *Invest. Ophthalmol. Vis. Sci.*, *25*, 268 (1984). The results of the determination expressed as percent decrease of outflow pressure is presented in the Table.

TABLE

| COMPOUND | CONCENTRATION (%) | DECREASE IN OUT-FLOW PRESSURE (%) |
|---|---|---|
| 7ß-acetoxy-8,13-epoxy-1α-diethylaminoacetoxy-6ß,9α-dihydroxylabd-14-en-11-one | 1.0 | 66 |
| | 0.1 | 30 |
| 7ß-acetoxy-8,13-epoxy-1α-(morpholin-4-yl)acetoxy-6ß,9α-dihydroxylabd-14-en-11-one | 1.0 | 63 |
| 7ß-acetoxy-8,13-epoxy-1α,6ß,9α-trihydroxylabd-14-en-11-one | 1.0 | 51 |
| | 0.1 | 23 |

Intraocular pressure reduction is achieved when the present aminoacyllabdanes are administered to a

subject requiring such treatment as an effective topical dose of a 0.01 to 3.0% solution or suspension. A particularly effective amount is about 3 drops of a 1% preparation per day. It is to be understood, however, that for any particular subject, specific dosage regimens should be adjusted according to the individual need and professional judgment of the person administering or supervising the administration of the aforesaid compound. It is to be further understood that the dosages set forth herein are exemplary only and that they do not, to any extent, limit the scope or practice of the invention.

Compounds of the present invention include:

(a) 7β-Acetoxy-1α-(2-aminopropionyloxy)-8,13-epoxy-6β-9α-dihydroxylabd-14-en-11-one;

(b) 7β-Acetoxy-1α-[2-(N-benzylamino)propionyloxy)-8,13-epoxy-6β-9α-dihydroxylabd-14-en-11-one;

(c) 7β-Acetoxy-8,13-epoxy-1α-[2-(N-ethylamino)propionyloxy]-6β-9α-dihydroxylabd-14-en-11-one;

(d) 7β-Acetoxy-8,13-epoxy-1α-[2-(N-ethylamino)-3-phenylpropionyloxy]-6β-9α-dihydroxylabd-14-en-11-one;

(e) 6β-Acetoxy-8,13-epoxy-7β-9α-dihydroxy-1α-(dimethylaminoacetoxy)labd-14-en-11-one;

(f) 7β-Acetoxy-8,13-epoxy-6β-formyloxy-9α-hydroxy-1α-(dimethylaminoacetoxy)labd-14-en-11-one;

(g) 8,13-Epoxy-7β-formyloxy-6β,9α-dihydroxy-1α-(dimethylaminoacetoxy)labd-14-en-11-one;

(h) 8,13-Epoxy-6β,7β,9α-trihydroxy-1α-(dimethylaminoacetoxy)labd-14-en-11-one carbonate;

(i) 8,13-Epoxy-6β,7β,9α-trihydroxy-1α-(dimethylaminoacetoxy)labd-14-en-11-one sulfite.

The aminoacyllabdanes of the present invention are also useful in the treatment of hypertension, congestive heart failure, bronchial asthma and psoriasis.

Effective amounts of the compounds of the present invention may be administered to a subject by any one of various methods, for example, orally as in capsules or tablets, parenterally in the form of sterile solutions or suspensions, in some cases intravenously in the form of sterile solutions, or suspension, and topically in the form of solutions, suspensions or ointments, and by aerosol spray. The aminoacyllabdanes of the present invention, while effective themselves, may be formulated and administered in the form of their pharmaceutically acceptable addition salts for purposes of increased solubility and the like.

Preferred pharmaceutically acceptable addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example citric acid and the like.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 0.1—30 milligrams of the active compound.

The tablets, pills, capsules, troches and the like may also contain the following ingredients: a binder such as microcrystalline cellulose, gum tragancanth or gelatin; an excipient such as starch or lactose, a disintegrating agent such as alginic acid, corn starch and the like; a lubricant such as magnesium stearate; a glidant such as colloidal silicon dioxide; and a sweetening agent such as sucrose or saccharin or a flavoring agent such as peppermint, methyl salicylate, orange flavoring may be added. When the dosage unit form is a capsule, it may contain, in addition to materials of the above type, a liquid carrier such as a fatty oil. Other dosage unit form may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral or topical therapeutic administration, the active compounds of the invention may be incorporated into a solution, suspension, ointment or cream. These preparations should contain at least 0.01% of active compound, but may be varied between 0.5 and about 5% of the weight thereof. The amount of active compounds in such compositions is such that a suitable dosage will be obtained. Preferred compositions and preparations according to the present invention are prepared so that a parenteral or topical dosage unit contains 0.01 to 10 milligrams of active compound.

The solutions or suspensions for topical or parenteral administration may also include the following components: a sterile diluent such as water for injection, saline solution, fixed oils, polyethylene glycols, glycerine, propylne glycol or other synthetic solvents; antibacterial agents such as benzyl alcohol or methyl parabens; antioxidants such as ascorbic acid or sodium bisulfite; chelating agents such as ethylendiaminetetraacetic acid; buffers such as acetates, citrates or phosphates and agents for the adjustment of tonicity such as sodium chloride or dextrose. The parenteral preparation can be enclosed in ampules or disposable syringes; the topical preparation may be enclosed in multiple dose vials or dropping bottles, made of glass or plastic.

All temperatures are given in degrees Centigrade.

Example 1

7β-Acetoxy-8,13-epoxy-1α-(diethylaminoacetoxy)-6β-9α-dihydroxylabd-14-en-11-one hydrochloride

To 200 g of 7β-acetoxy-8,13-epoxy-1α,6β-9α-trihydroxylabd-14-en-11-one was added a solution of 0.70 ml of N,N-dimethylaniline in 1 ml of dry (3Å molecule sieves) dichloromethane. The solution, under nitrogen, was cooled in an ice bath. To the solution was added slowly, dropwise, a solution of 0.050 ml of bromoacetyl bromide. The mixture was stirred 2 hrs at ice bath temperature and 1 hr at room temperature. The mixture was diluted with ethyl acetate, washed with sodium bicarbonate solution and water, and dried over anhydrous sodium sulfate. Filtration followed by evaporation of the solvent provided an oil. The oil was dissolved in 3 ml of ethyl acetate. To the solution was added 0.068 mg of sodium bicarbonate and a solution of 0.05 ml of diethylamine in 1 ml of ethyl acetate. The mixture was stirred 0.5 hr at 80°, allowed to cool to room temperature and filtered. The filtrate was diluted with ethyl acetate, washed with water, saturated sodium chloride solution and dried over anhydrous sodium sulfate. Filtration followed by evaporation of solvent provided an oil. The oil was dissolved in a minimum volume of 70/30 hexane/ethyl acetate and chromatographed on 50 g of silica gel (230—400 mesh, nitrogen pressure, eluent: 30 × 15 ml of 70/30 hexane/ethyl acetate). The appropriate fractions were concentrated and the solvent evaporated to provide 129 mg (50.5%) of product as an oil. The hydrochloride was precipitated/crystallized from ether to provide product as the hydrochloride, mp 160—165°C.

ANALYSIS:

| | | | |
|---|---|---|---|
| Calculated for $C_{28}H_{45}NO_8HCl$: | 60.04 %C | 8.28 %H | 2.50 %N |
| Found: | 59.79 %C | 7.93 %H | 2.44 %N |

Example 2

7β-Acetoxy-8,13-epoxy-1α-(dimethylaminoacetoxy)-6β-9α-dihydroxylabd-14-en-11-one hydrochloride

To 100 g of 7β-acetoxy-8,13-epoxy-1α,6β-9α-trihydroxylabd-14-en-11-one was added a solution of 0.035 ml of N,N-dimethylaniline in 1 ml of dry dichloromethane. The solution was cooled in an ice bath. To the solution was added slowly, dropwise, a solution of 0.025 ml of bromoacetyl bromide in 1 ml of dry dichloromethane. The mixture was stirred 1 hr at ice bath temperature and allowed to warm to room temperature. The mixture was diluted with ethyl acetate, washed with sodium bicarbonate solution, water and dried over anhydrous sodium sulfate. Filtration followed by evaporation of the solvent provided an oil. The oil was dissolved in 2 ml of ethyl acetate. To the solution was added 1 ml of ethyl acetate which had been saturated with dimethylamine (gas). The solution was stirred at room temperature for 15 min., diluted with ethyl acetate, washed with sodium bicarbonate solution, saturated sodium chloride solution and dried over anhydrous sodium sulfite. Filtration followed by evaporation of solvent provided an oil. The oil was chromatographed on silica gel (20 g, 230—400 mesh; eluent: 50 × 15 ml of 70/30 hexane/ethyl acetate). Evaporation of the appropriate fractions provided 93 mg (76.6%) of product as an oil, which upon addition of etheral hydrogen chloride, precipitated the hydrochloride, mp. 156—182°.

ANALYSIS:

| | | | |
|---|---|---|---|
| Calculated for $C_{26}H_{41}NO_8HCl$: | 58.69 %C | 7.96 %H | 2.63 %N |
| Found: | 58.55 %C | 8.06 %H | 2.56 %N |

Example 3

7β-Acetoxy-8,13-epoxy-1α-[(pyrrolidin-1-yl)acetoxy]-6β-9α-dihydroxylabd-14-en-11-one hydrochloride

To a stirred solution of 100 g of 7β-acetoxy-8,13-epoxy-1α,6β-9α-trihydroxylabd-14-en-11-one in 1 ml of dry dichloromethane and 0.035 l of N,N-dimethylaniline at 0°, under nitrogen, was added dropwise a solution of 0.025 ml of bromoacetyl bromide in 1 ml of dry dichloromethane. The mixture was allowed to warm to room temperature, diluted with ethyl acetate, washed with sodium bicarbonate solution, water and dried over anhydrous sodium sulfate. The solution was filtered and concentrated to an oil. The oil was dissolved in 3 ml of ethyl acetate and added to a stirred solution of 0.1 g of pyrrolidine in 1 ml of ethyl acetate. The mixture was stirred 0.5 hr at room temperature, diluted with ethyl acetate, extracted twice with ethyl acetate, washed three times with water, once with saturated sodium chloride solution, dried over sodium sulfate, filtered and the solvent evaporated to provide an oil. The oil was dissolved in a minimum volume of 70% ethyl acetate/hexane, and flash chromatographed on 50 g of silica gel (230—400 mesh; 75 × 15 ml). Concentration of the appropriation fractions provided an oil. The oil was dissolved in anhydrous ether and the hydrochloride precipitated to provide 50.5 mg (37.1%) of product, mp 148—158.

ANALYSIS:

| | | | |
|---|---|---|---|
| Calculated for $C_{28}H_{43}NO_8HCl$: | 60.25 %C | 7.95 %H | 2.51 %N |
| Found: | 60.64 %C | 8.29 %H | 2.50 %N |

Example 4

7β-Acetoxy-8,13-epoxy-1α-[(morpholin-4-yl)acetoxy]-6β-9α-dihydroxylabd-14-en-11-one hydrochloride

To a stirred solution of 109 mg of 7β-acetoxy-8,13-epoxy-1α,6β-9α-trihydroxylabd-14-en-11-one in 1 ml of dry dichloromethane containing 0.038 ml of dimethylamiline in an ice bath was added dropwise a

solution of 0.029 ml bromoacetyl bromide in one ml of dry dichlorometha... The mixture was stirred at ice bath temperature for one hour, diluted with ethyl acetate, washed with water, saturated sodium bicarbonate solution, water and dried over anhydrous sodium sulfate. Filtration followed by evaporation of solvent provided an oil which was dissolved in 2 ml of dry dichloromethane and added to a solution of 0.1 g of morpholine in 2 ml of ethyl acetate. The mixture was stirred 1 hr at room temperature, diluted with ethyl acetate, washed with water, saturated sodium bicarbonate solution, water and dried over anhydrous sodium sulfate. The solution was filtered and concentrated to an oil. The oil was dissolved in a minimum volume of 1/1 ethyl acetate/hexane and flash chromatographed on silica gel (230—400 mesh) (eluent: 20 × 15 ml of 1/1 ethyl acetate/hexane). The appropriate fractions were combined and concentrated to an oil. The oil was dissolved in ether and precipitated with ethereal hydrogen chloride to provide 67.3 mg (61.7%) of product as the hydrochloride, mp 153—163°.

ANALYSIS:

| | | | |
|---|---|---|---|
| Calculated for $C_{25}H_{43}NO_9HCl$: | 58.57 %C | 7.73 %H | 2.44 %N |
| Found: | 58.99 %C | 7.69 %H | 2.14 %N |

Example 5

7β-Acetoxy-8,13-epoxy-1α-[(di-n-propylamino)acetoxy]-6β,9α-dihydroxylabd-14-en-11-one hydrochloride

To a stirred solution of 108 mg of 7β-acetoxy-8,13-epoxy-1α,6β-9α-trihydroxylabd-14-en-11-one in a solution of 1 ml of dry dichloromethane containing 0.038 ml of dimethylaniline, in an ice bath, under nitrogen, was added slowly dropwise a solution of 0,027 ml of bromoacetyl bromide in 1 ml of dry dichloromethane. The mixture was stirred at 0° for 1 hr, allowed to warm to room temperature, diluted with ethyl acetate, washed with cold water, saturated sodium bicarbonate solution and dried over anhydrous sodium sulfate. Filtration followed by evaporation of solvent provided an oil. The oil was dissolved in 1 ml of dichloromethane and added to a stirred solution of 0.1 g of di-n-propylamine in 1 ml of ethyl acetate. The solution was stirred 1 hr at room temperature and extracted with ether. The extracts were washed twice with water, once with saturated sodium chloride solution and dried over anhydrous sodium sulfate. Filtration followed by evaporation of solvent provided an oil. The oil was dissolved in a minimum volume of 20% ethyl acetate/hexane and flash chromatographed on 25 g of silica gel (230—400 mesh) (eluent: 25 × 10 ml of 20% ethyl acetate/hexane). Concentration of the appropriate fractions provided an oil. Addition of ethereal hydrogen chloride to an ethereal solution of the oil provided 59.9 mg (38.8%) of product as the hydrochloride, mp 199—202°.

ANALYSIS:

| | | | |
|---|---|---|---|
| Calculated for $C_{30}H_{49}NO_8HCl$: | 61.26 %C | 8.57 %H | 2.38 %N |
| Found: | 61.08 %C | 8.46 %H | 2.26 %N |

Example 6

7β-Epoxy-1α-(diethylaminoacetoxy)-6β,7β,9α-trihydroxylabd-14-en-11-one-hydrochloride

A solution of 200 mg of 7β-acetoxy-8,13-epoxy-1α,6β,9α-trihydroxylabd-14-en-11-one in 10 ml of a saturated potassium carbonate solution in 20% aqueous methanol was stirred 2 hrs at 25—28°. The mixture was diluted with ethyl acetate, washed twice with water, once with saturated sodium chloride solution, dried over anhydrous sodium sulfate, filtered and concentrated to provide 157 ml of 8,13-epoxy-1α,6β,7β,9α-tetrahydroxylabd-14-en-11-one as an oil.

To a stirred solution of 157 mg of 8,13-epoxy-1α,6β,7β,9α-tetrahydroxylabd-14-en-11-one, 0.0593 ml of N,N-dimethylaniline and 1 ml of dry dichloromethane in an ice bath, under nitrogen, was added dropwise a solution of 0.0421 ml of bromoacetyl bromide in 1 ml of dry dichloromethane. The mixture was stirred 1 hr at 0°, poured into ice water/ethyl acetate, washed with cold saturated sodium bicarbonate solution, water and dried over anhydrous sodium sulfate. Filtration followed by evaporation of solvent provided an oil. The oil was dissolved in 1 ml of dry dichlormethane and added to a stirred solution of 0.1 g of diethylamine 1 ml of ethyl acetate. The solution was stirred for 1 hr at ambient temperature and extracted with ethyl acetate. The extract was washed with water, saturated sodium chloride solution and dried over anhydrous sodium sulfate. Filtration followed by evaporation provided an oil. The oil was dissolved in a minimum volume of 1/1 ethyl acetate/hexane and flash chromatographed on 20 g of silica gel (230—4000 mesh) (eluent: 25 × 10 ml of 1/1 ethyl acetate/hexane). Concentration of the appropriate fractions provided an oil, the hydrochloride of which was precipitated from ether to yield 90.2 mg (42.4%) of product, mp 127—157°C.

ANALYSIS:

| | | | |
|---|---|---|---|
| Calculated for $C_{26}H_{43}NO_7HCl$: | 60.27 %C | 8.56 %H | 2.70 %N |
| Found: | 59.99 %C | 8.74 %H | 2.58 %N |

Example 7

7β-Acetoxy-8,13 epoxy-1α-[(4-hydroxypiperidin-1-yl)acetoxy]-6β,9α-dihydroxylabd-14-en-11-one-hydrochloride

To a stirred solution of 106 mg of 7β-acetoxy-8,13-epoxy-1α,6β,9α-trihydroxylabd-14-en-11-one in 1 ml

of dry dichloromethane containing a 0.038 ml of dimethylaniline, in an ice bath, was added dropwise a solution of 0.027 ml of bromoacetyl bromide in 1 ml of dry dichloromethane. The mixture was stirred at 0° for 1 hr, allowed to warm to room temperature, poured onto ice sodium bicarbonate ethyl acetate, extracted with ethyl acetate, washed with water and dried over anhydrous sodium sulfate. Filtration followed by evaporation of solvent provided an oil which has dissolved in 1 ml of dichloromethane and added to a stirred solution of 0.10 g of 4-hydroxypiperidine in 1 ml of ethyl acetate. The mixture was stirred 1 hr at room temperature, poured onto ice/water ethyl acetate, extracted with ethyl acetate, washed with water, saturated sodium chloride solution and dried over anhydrous sodium sulfate. Filtration followed by evaporation of the solvent provided an oil. The oil was dissolved in a minimum volume of 30% ethyl acetate/hexane and flash chromatographed on 25 g of silica gel (230—400 mesh). Concentration of the appropriate fractions gave an oil. Treatment of the oil with ethereal hydrogen chloride gave 0.109 g (76.4%) of product as the hydrochloride, mp 166—189°.

Example 8

7β-Acetoxy-8,13 epoxy-1α-[(thiomorpholin-4-yl)acetoxy]-6β,9α,-dihydroxylabd-14-en-11-one

To a stirred solution of 104 mg of 7β-acetoxy-8,13-epoxy-1α,6β,9α-trihydroxylabd-14-en-11-one containing 0.037 ml of N,N-dimethylaniline in 1 ml of dry dichloromethane at 0° under nitrogen was added dropwise a solution of 0.025 ml of bromoacetyl bromide. The mixture was stirred 1 hr at 0°, allowed to warm to room temperature, poured into ice, extracted with ethyl acetate, washed with anhydrous sodium sulfate, filtered, and the filtrate concentrated to an oil. The oil was dissolved in 1 ml of dry dichloromethane and the solution was added dropwise to a solution of 0.1 g of thiomorpholine in 1 ml of ethyl acetate. The mixture was stirred 1.5 hr, poured onto ice, diluted with ethyl acetate, washed with water, saturated sodium chloride and dried over anhydrous sodium sulfate. Filtration followed by evaporation of solvent and purification by flash chromatography to yield an oil. The oil was dissolved in ether and ethereal hydrogen chloride was added to provide 69.1 mg (47.8%) of product, as the hydrochloride, mp 161—171°.

Example 9

7β-Acetoxy-8,13 epoxy-1α-[(piperidin-1-yl)acetoxy]-6β,9α-dihydroxylabd-14-en-11-one-hydrochloride

To a stirred solution of 104 mg of 7β-acetoxy-8,13-epoxy-1α,6β,9α-trihydroxylabd-14-en-11-one in 1 ml of dry dichloromethane containing under 0.037 ml of dimethylaniline in an ice bath, was added slowly, dropwise, a solution of 0.026 ml of bromoacetyl bromide in 1 ml of dry dichloromethane. The mixture was stirred for 1 hr at ice bath temperature, allowed to warm to room temperature, diluted with ethyl acetate, poured into ice/water, extracted with ethyl acetate, washed with cold, saturated sodium bicarbonate solution, water, and dried over anhydrous sodium sulfate. Filtration followed by evaporation of the solvent provided an oil which was dissolved in 1.5 ml of dichlormethane and added dropwise to a stirred solution of 0.1 g of piperidine in 1 ml of ethyl acetate. The mixture was stirred for 1.5 hr at room temperature. The solution was diluted with ethyl acetate, washed with water, saturated sodium chloride solution and dried over anhydrous sodium sulfate. Filtration followed by evaporation of solvent provided an oil. The oil was purified by flash chromatography on silica gel (230—400 mesh; eluent: 30% ethyl acetate/hexane). Evaporation of the solvent provided an oil, which was treated with ethereal hydrogen chloride to provide 34.8 mg (23.9%) of product, as the hydrochloride, mp 160—184°.

ANALYSIS:

| | | | |
|---|---|---|---|
| Calculated for $C_{28}H_{45}NO_8HCl$: | 60.88 %C | 8.10 %H | 2.45 %N |
| Found: | 60.29 %C | 7.93 %H | 2.24 %N |

Example 10

7β-Acetoxy-8,13 epoxy-1α-(isopropylaminoacetoxy)6β,9α-dihydroxylabd-14-en-11-one-hydrochloride

To a stirred solution of 0.0301g of 7β-acetoxy-8,13-epoxy-1α,6β,9α-trihydroxylabd-14-en-11-one in a stirred solution of 3 ml of dry dichlormethane containing 0.106 ml of dimethylaniline at 0°, under nitrogen, was added dropwise a solution of 0.075 ml of bromoacetyl bromide in 3 ml of dry dichloromethane. The mixture was stirred 1 hr at 0°, poured into ice water, washed with ice cold saturated sodium bicarbonate solution, water and dried over anhydrous sodium sulfate. Filtration followed by evaporation of solvent provided an oil. The oil was dissolved in 3 ml of dry dichloromethane and added dropwise to a stirred solution of 0.300 g of isopropylamine in 3 ml of ethyl acetate. The mixture was stirred 2.5 hr at room temperature, poured into ice water, extracted with ethyl acetate, washed with saturated sodium chloride solution and dried over anhydrous sodium sulfate. Filtration followed by evaporation of solvent provided an oil which was flash chromatographed on 20 g of silica gel (230—400 mesh) (eluent( 70% ethyl acetate/hexane 0.1% ammonium hydroxide). Concentration of the appropriate fractions provided an oil. Treatment of the oil with ethereal hydrogen provided under 0.245 g (61.3%) of product, as the hydrochloride, mp 154—174°.

ANALYSIS:

| | | | |
|---|---|---|---|
| Calculated for $C_{27}H_{43}NO_7HCl$: | 59.38 %C | 8.12 %H | 2.57 %N |
| Found: | 58.82 %C | 8.05 %H | 2.74 %N |

Example 11

7β-Acetoxy-8,13 epoxy-1α-(t-butylamino)acetoxy-6β,9α-dihydroxylabd-14-en-11-one-hydrochloride

To a stirred solution of 106 mg of 7β-acetoxy-8,13-epoxy-1α,6β,9α-trihydroxylabd-14-en-11-one and 0.036 ml of dimethylaniline in 1 ml of dry dichloromethane in an ice bath was added dropwise a solution of 0.025 ml of bromoacetyl bromide in 1 ml of dry dichloromethane. The mixture was stirred 1 hr at ice bath temperature. The reaction mixture was allowed to warm to room temperature, diluted with ethyl acetate, poured onto ice/water, extracted with ethyl acetate, washed with cold saturated sodium bicarbonate solution, water and dried over anhydrous sodium sulfate. Filtration followed by evaporation of the solvent provided an oil. The oil was dissolved in 1 ml of dichloromethane and added dropwise to a solution of 0.105 g of t-butylamine in 1 ml of ethyl acetate. The mixture was stirred at room temperature for 15 hrs, diluted with ethyl acetate, washed with water, saturated sodium chloride solutions and dried over anhydrous sodium sulfate. Filtration followed by evaporation of the solvent provided an oil. The oil was purified by flash chromatography (230—400 mesh silica gel; eluent: 30% ethyl acetate/hexane). Concentration of the appropriate fractions provided an oil, which was treated with ethereal hydrogen chloride, to provide 69.9 mg (49.3%) of product, as the hydrochloride, mp 165—179°.

ANALYSIS:

| | | | |
|---|---|---|---|
| Calculated for $C_{19}H_{45}NO_8HCl$: | 60.04 %C | 8.28 %H | 2.50 %N |
| Found: | 59.73 %C | 8.22 %H | 2.26 %N |

Example 12

7β-Acetoxy-8,13-epoxy-1α-[2-(ethylamion)propionyloxy]-6β,9α-dihydroxylabd-14-en-11-one hydrochloride

To a stirred solution of 1.0 g of 7β-acetoxy-8,13-epoxy-1α,6β,9α-trihydroxylabd-14-en-11-one in 10 ml of dichloromethane was added 0.35 ml of N,N-dimethylaniline. To the mixture was added slowly, by syringe, a solution of 0.30 ml (0.618 g) of bromopropionyl bromide in 10 ml of dichloromethane. The mixture was stirred 1 hr at room temperature, cooled in an ice-bath, poured into ice/water/sodium bicarbonate and the mixture was extracted with ethyl acetate. The extracts were washed with water, dried over anhydrous sodium sulfate, filtered and concentrated to an oil. The oil was dissolved in 10 ml of dichloromethane and added by syringe to a stirred solution of excess monoethylamine in 10 ml of ethyl acetate in an ice-bath. The solution was allowed to warm to room temperature and stirred for 3 hrs. The solution was poured into ice/water/ethyl acetate and extracted twice with ethyl acetate. The extracts were washed twice with water, with saturated sodium chloride solution, dried over anhydrous sodium sulfate and filtered. Filtration followed by evaporation of the solvent provided an oil. The oil was dissolved in a minimum volume of 2% methanol/dichloromethane and flash chromatographed on 150 g of silica gel (230—400 mesh). The column was eluted as follows: 1 × 400 ml 2% methanol/dichloromethane, 1 × 200 ml 2% methanol/dichloromethane.

3 × 100 ml 4% methanol/dichloromethane, 10 × 50 ml 4% methanol dichloromethane. A diasteriomer was contained in the first two 4% methanol/dichloromethane fractions. A mixture of diasteriomers was contained in the third 4% methanol/dichlormethane fraction. A second diasteriomer was contained in fractions 4 through 6. The diasteriomers were isolated. The diasteriomers were then combined, concentrated to an oil, dissolved in anhydrous ether and precipitated with ethereal hydrogen chloride to provide 0.354 g (26.6%) of product, mp 160—174°.

ANALYSIS:

| | | | |
|---|---|---|---|
| Calculated for $C_{27}H_{43}NO_8 \cdot HCl$: | 59.38 %C | 8.12 %H | 2.57 %N |
| Found: | 59.00 %C | 7.94 %H | 2.39 %N |

Example 13

7β-Acetoxy-8,13-epoxy-1α-[2-(morpholin-4-yl)propionyloxy]-6β,9α-dihydroxylabd-14-en-11-one hydrochloride

To a stirred solution of 0.3 g of 7β-acetoxy-8,13-epoxy-1α,6β,9α-trihydroxylabd-14-en-11-one in 3 ml of dichloromethane under nitrogen was added 0.106 m of dimethylaniline. The solution was cooled to 0° and a solution of 0.090 ml of 2-bromopropionyl bromide in 3 ml of dichloromethane was added dropwise. The solution was stirred 1 hr at 0°, poured into ice/sodium bicarbonate/ethyl acetate and the mixture was extracted twice with ethyl acetate. The extracts were washed with water, dried over anhydrous sodium sulfate and filtered. Filtration followed by evaporation of solvent provided an oil. The oil was dissolved in 3 ml of dichloromethane and added to a stirred solution of 0.3 g of morpholine in 3 ml of ethyl acetate.

The solution was stirred at room temperature for 3 hr, poured into water/ice/ethyl acetate and the mixture was extracted twice with ethyl acetate. The extracts were washed twice with water and once with sodium chloride solution. The solution was dried over anhydrous sodium sulfate, filtered and concentrated to an oil. The oil was dissolved in a minimum volume of 40% ethyl acetate/hexane and flash chromatographed on 150 g of silica gel (eluent: 10 × 50 ml of 40% ethyl acetate/hexane). Individual fractions were concentrated and the two diasteriomers were isolated. The fractions containing either diasteriomer were then combined and the solvent was evaporated. The resultant oil was dissolved in ether

and precipitated with ethereal hydrogen chloride to provide, after drying, 0.121 g (28%) of product, mp 160—170°C.

ANALYSIS:

| | | | |
|---|---|---|---|
| Calculated for $C_{29}H_{45}NO_9 \cdot HCl$: | 59.22 %C | 7.88 %H | 2.38 %N |
| Found: | 59.53 %C | 7.93 %H | 2.17 %N |

Example 14

7β-Acetoxy-8,13-epoxy-1α-(4-methylpiperazin-1-yl)acetoxy-6β,9α-dihydroxylabd-14-en-11-one hydrochloride

To a stirred solution of 300 ml of 7β-acetoxy-8,13-epoxy-1α,6β,9α-trihydroxylabd-14-en-11-one in 3 ml of dichloromethane containing 0.106 ml of dimethylaniline at 0° was added dropwise a solution of 0.075 ml (0.175 g) of bromoacetyl bromide in 3 ml of dichloromethane. The mixture was stirred 1 hr at 0°, poured into ice/saturated sodium bicarbonate solution/ethyl acetate, and the mixture was extracted with ethyl acetate. The extracts were washed with water, dried over anhydrous sodium sulfate and filtered. Filtration followed by evaporation of the solvent provided an oil. The oil was dissolved in dichloromethane and added to a solution of 0.3 g of N-methylpiperazine in 3 ml of ethyl acetate. The mixture was stirred 2 hrs at room temperature, poured onto ice/water/ethyl acetate and the layers were separated. The extracts were washed with water, saturated sodium chloride solution and dried over anhydrous sodium sulfate. Filtration followed by evaporation of solvent provided an oil. The oil was dissolved in a minimum volume of 7% methanol/dichloromethane/0.1% ammonium hydroxide and flash chromatographed on silica gel (230—400 mesh), eluent: 7% methanol/dichloromethane/0.1%, ammonium hydroxide, followed by 10% methanol/dichloromethane/0.1% ammonium hydroxide. Evaporation of solvent from the appropriate fractions provided an oil, which was dissolved in 5% ethyl acetate/ether and precipitated with ethereal hydrogen chloride to provide 0.242 g (56.3%) of product, mp 189—196° dec.

ANALYSIS:

| | | | |
|---|---|---|---|
| Calculated for $C_{29}H_{46}N_2O_8 \cdot HCl$: | 59.32 %C | 8.07 %H | 4.77 %N |
| Found: | 59.12 %C | 8.02 %H | 4.77 %N |

REACTION SCHEME

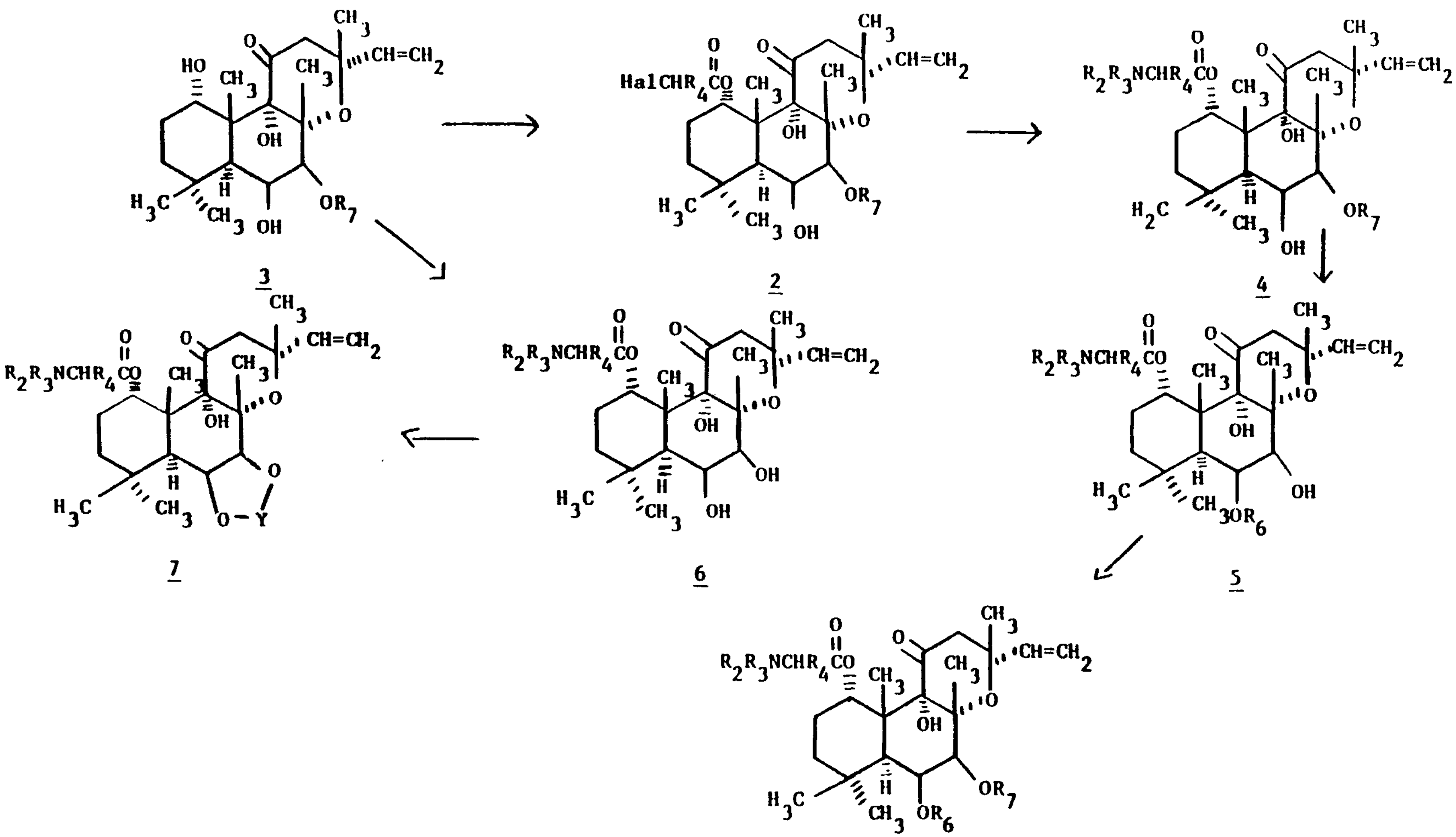

3

2

4

7

6

5

8

wherein $R_2$, $R_3$, $R_4$, $R_6$, $R_7$, Hal and Y are as hereinbeforedescribed.

**Claims for the Contracting States: BE CH DE FR GB IT LI LU NL SE**

1. A compound of the formula

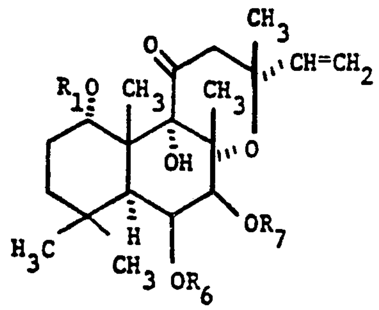

wherein $R_1$ is $R_2R_3NCHR_4CO$ wherein $R_2$ is hydrogen, $C_1$—$C_6$-alkyl or benzyl, $R_3$ is hydrogen or $C_1$—$C_6$-alkyl and $R_4$ is hydrogen, $C_1$—$C_6$-alkyl or benzyl or $R_2$ and $R_3$ taken together with the nitrogen atom to which they are attached form a group of the formula

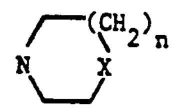

wherein X is CO, O, S, SO, $SO_2$; a group of the formula $CHR_9$ wherein $R_9$ is hydrogen, $C_1$—$C_6$-alkyl or a group of the formula $OR_{10}$ wherein $R_{10}$ is hydrogen or $COR_{11}$ wherein $R_{11}$ is $C_1$—$C_6$-alkyl; or a group of the formula $NR_{12}$ wherein the $R_{12}$ is $C_1$—$C_6$-alkyl; n is 1 or 0; $R_6$ is hydrogen or a group of the formula $R_5CO$ wherein $R_5$ is hydrogen or $C_1$—$C_6$-alkyl; $R_7$ is hydrogen or a group of the formula $R_8CO$ wherein $R_8$ is hydrogen or $C_1$—$C_6$-alkyl or $R_6$ and $R_7$ taken together form a group of the formula CO or SO; the optical and geometric isomers thereof, or a pharmaceutically acceptable acid addition salt thereof.

2. A compound according to claim 1 wherein $R_2$ and $R_3$ are both $C_1$—$C_6$-alkyl.

3. A compound according to claim 1 wherein $R_2$ and $R_3$ taken together with the nitrogen atom to which they are attached form a group of the formula

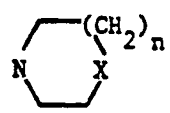

wherein X is O, S, a group of the formula $CHR_9$ wherein $R_9$ is hydrogen or a group of the formula $OR_{10}$ wherein $R_{10}$ is hydrogen and n is 0 or 1.

4. A compound according to any of claims 2 or 3, wherein $R_4$ is hydrogen or $C_1$—$C_6$-alkyl.

5. A compound according to any of claims 2, 3 or 4 wherein $R_6$ is hydrogen and $R_7$ is a group of the formula $R_8CO$ where $R_8$ is hydrogen or $C_1$—$C_6$-alkyl.

6. A compound of the formula

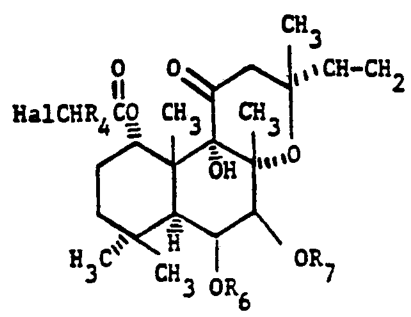

2

wherein $R_4$ is hydrogen, lower alkyl or benzyl; $R_6$ is hydrogen or a group of the formula $R_5CO$ wherein $R_5$ is hydrogen or $C_1$—$C_6$-alkyl; $R_7$ is hydrogen or a group of the formula $R_8CO$ wherein $R_8$ is hydrogen or $C_1$—$C_6$-alkyl; Hal is chloro or bromo; or the optical and geometric isomers thereof.

7. A compound according to claim 6 wherein Hal is bromo.

8. A pharmaceutical composition which comprises as active ingredient a compound as claimed in claim 1 in association with a pharmacologically acceptable carrier.

9. Use of a compound as claimed in claim 1 for the preparation of a medicament having intraocular pressure reducing activity.

10. A process for the preparation of a compound of the formula

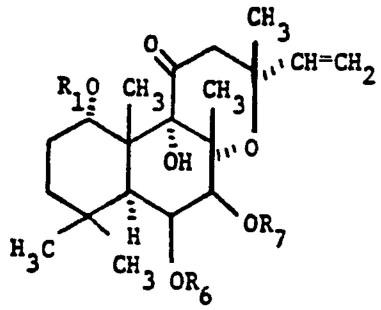

wherein $R_1$ is $R_2R_3NCHR_4CO$ wherein $R_2$ is hydrogen, $C_1$—$C_6$-alkyl or benzyl, $R_3$ is hydrogen or $C_1$—$C_6$-alkyl and $R_4$ is hydrogen, $C_1$—$C_6$-alkyl or benzyl; $R_2$ and $R_3$ taken together with the nitrogen atom to which they are attached form a group of the formula

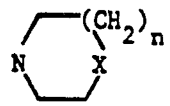

wherein X is CO, O, S, SO, $SO_2$, a group of the formula $CHR_9$ wherein $R_9$ is hydrogen, $C_1$—$C_6$-alkyl or a group of the formula $OR_{10}$ wherein $R_{10}$ is hydrogen or $COR_{11}$ wherein $R_5$ is $C_1$—$C_6$-alkyl, or a group of the formula $NR_{12}$ wherein the $R_{12}$ is $C_1$—$C_6$-alkyl; n is 0 or 1; $R_6$ is hydrogen or a group of the formula $R_5CO$ wherein $R_5$ is hydrogen or $C_1$—$C_6$-alkyl; $R_7$ is hydrogen or a group of the formula $R_8CO$ wherein $R_8$ is hydrogen or $C_1$—$C_6$-alkyl; and $R_6$ and $R_7$ taken together form a group of the formula CO or SO; the optical and geometric isomers thereof, or a pharmaceutically acceptable acid addition salt thereof, which comprises:

(a) contacting a compound of the formula

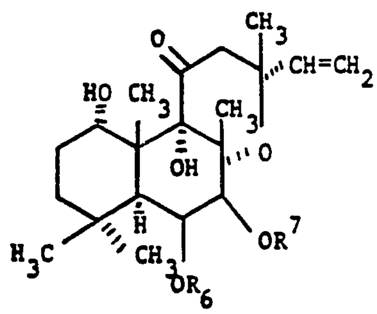

wherein $R_6$ and $R_7$ are as described above with a compound of the formula

$HalCHR_4COHal$

wherein $R_4$ is as described above and Hal is bromo or chloro in the presence of a tertiary amine to provide a compound of the formula

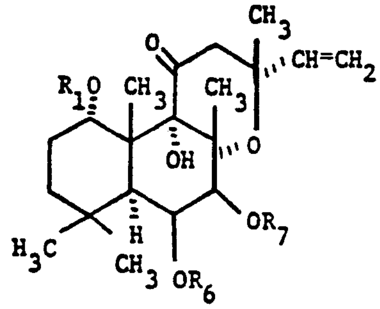

wherein $R_1$ is $HalCHR_4CO$ wherein $R_4$ and Hal are as above; and

(b) contacting the product of step (a) with a compound of the formula $R_2R_3NH$ wherein $R_2$ and $R_3$ are as above.

**Claims for the Contracting State: AT**

1. A process for the preparation of a compound of the formula

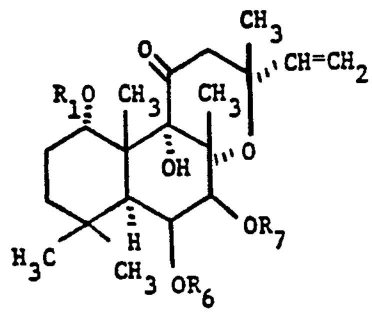

wherein $R_1$ is $R_2R_3NCHR_4CO$ wherein $R_2$ is hydrogen, $C_1$—$C_6$-alkyl or benzyl, $R_3$ is hydrogen or $C_1$—$C_6$-alkyl and $R_4$ is hydrogen, $C_1$—$C_6$-alkyl or benzyl; $R_2$ and $R_3$ taken together with the nitrogen atom to which they are attached form a group of the formula

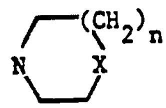

wherein X is CO, O, S, SO, $SO_2$, a group of the formula $CHR_9$ wherein $R_9$ is hydrogen, $C_1$—$C_6$-alkyl or a group of the formula $OR_{10}$ wherein $R_{10}$ is hydrogen or $COR_{11}$ wherein $R_{11}$ is $C_1$—$C_6$-alkyl, or a group of the formula $NR_{12}$ wherein the $R_{12}$ is $C_1$—$C_6$-alkyl; n is 0 or 1; $R_6$ is hydrogen or a group of the formula $R_5CO$ wherein $R_5$ is hydrogen or $C_1$—$C_6$-alkyl; $R_7$ is hydrogen or a group of the formula $R_8CO$ wherein $R_8$ is hydrogen or $C_1$—$C_6$-alkyl; and $R_6$ and $R_7$ taken together form a group of the formula CO or SO; the optical and geometric isomers thereof, or a pharmaceutically acceptable acid addition salt thereof, which comprises:

(a) contacting a compound of the formula

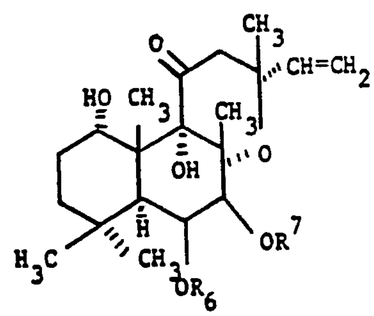

wherein $R_6$ and $R_7$ are as described above with a compound of the formula

$$HalCHR_4COHal$$

wherein $R_4$ is as described above and Hal is bromo or chloro in the presence of a tertiary amine to provide a compound of the formula

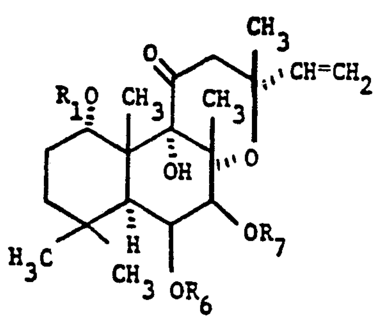

wherein $R_1$ is $HalCHR_4CO$ wherein $R_4$ and Hal are as above; and

(b) contacting the product of step (a) with a compound of the formula $R_2R_3NH$ wherein $R_2$ and $R_3$ are as above.

2. A process according to claim 1 wherein $R_2$ and $R_3$ are both $C_1$—$C_6$-alkyl.

3. A process according to claim 1 wherein $R_2$ and $R_3$ taken together with the nitrogen atom to which they are attached form a group of the formula

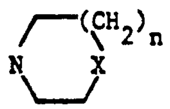

wherein X is O, S, a group of the formula $CHR_9$ wherein $R_9$ is hydrogen or a group of the formula $OR_{10}$ wherein $R_{10}$ is hydrogen and n is 0 or 1.

4. A process according to any of claims 2 or 3, wherein $R_4$ is hydrogen or $C_1$—$C_6$-alkyl.

5. A process according to any of claims 2, 3 or 4 wherein $R_6$ is hydrogen and $R_7$ is a group of the formula $R_8CO$ where $R_8$ is hydrogen or $C_1$—$C_6$-alkyl.

6. A process for the preparation of a compound of the formula *2*

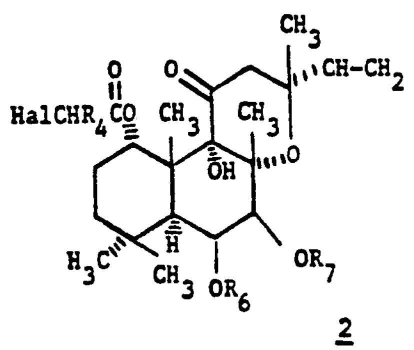

2

wherein Hal $R_4$, $R_6$ and $R_7$ are as defined in claim 1, which comprises treating a hydroxylabdane of the formula *3*

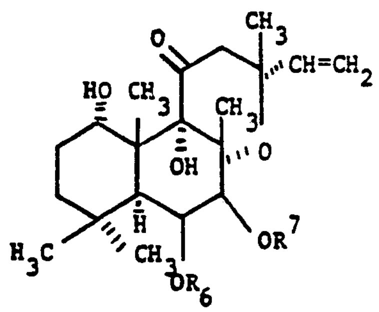

with a haloloweralkylcarbonyl halide of the formula $HalCHR_4COHal$, wherein Hal is bromo or chloro in the presence of a tertiary amine.

**Patentansprüche für die Vertragstaaten: BE CH DE FR GB IT LI LU NL SE**

1. Verbindung der Formel

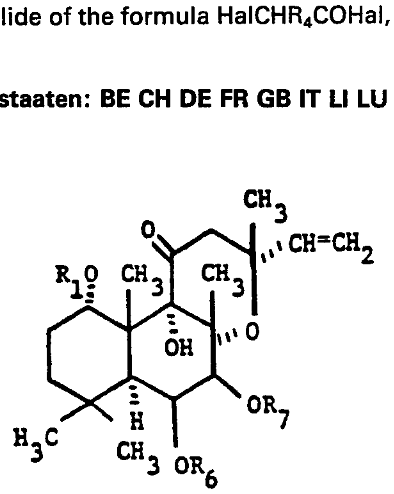

worin $R_1$ für $R_2R_3NCHR_4CO$ steht, worin $R_2$ Wasserstoff, $C_1$—$C_6$-Alkyl oder Benzyl bedeutet, $R_3$ Wasserstoff oder $C_1$—$C_6$-Alkyl bedeutet und $R_4$ Wasserstoff, $C_1$—$C_6$-Alkyl oder Benzyl ist, oder $R_2$ und $R_3$ zusammengenommen mit dem Stickstoffatom, an welches sie gebunden sind, eine Gruppe der Formel

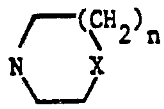

ausbilden, worin XCO, O, S, SO, $SO_2$, eine Gruppe der Formel $CHR_9$, worin $R_9$ Wasserstoff, $C_1$—$C_6$-Alkyl oder eine Gruppe der Formel $OR_{10}$ darstellt, worin $R_{10}$ für Wasserstoff oder $COR_{11}$ steht, worin $R_{11}$ $C_1$—$C_6$-Alkyl darstellt; oder eine Gruppe der Formel $NR_{12}$ bedeutet, worin $R_{12}$ $C_1$—$C_6$-Alkyl ist; n den Wert 1 oder 0 besitzt; $R_6$ für Wasserstoff oder eine Gruppe der Formel $R_5CO$ steht, worin $R_5$ Wasserstoff oder $C_1$—$C_6$-Alkyl bedeutet; $R_7$ Wasserstoff oder eine Gruppe der Formel $R_8CO$ darstellt, worin $R_8$ Wasserstoff oder $C_1$—$C_6$-Alkyl ist; oder $R_6$ und $R_7$ zusammengenommen eine Gruppe der Formel CO oder SO ausbilden; die optischen und geometrischen Isomeren hievon, oder ein pharmazeutisch annehmbares Säureadditionssalz hievon.

2. Verbindung nach Anspruch 1, worin $R_2$ und $R_3$ beide $C_1$—$C_6$-Alkyl darstellen.

3. Verbindung nach Anspruch 1, worin $R_2$ und $R_3$ zusammengenommen mit dem Stickstoffatom, an welches sie gebunden sind, eine Gruppe der Formel

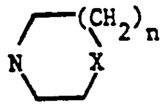

ausbilden, worin X für O, S oder eine Gruppe der Formel $CHR_9$ steht, worin $R_9$ Wasserstoff oder eine Gruppe der Formel $OR_{10}$ darstellt, worin $R_{10}$ Wasserstoff ist und n den Wert 0 der 1 besitzt.

4. Verbindung nach einem der Ansprüche 2 oder 3, worin $R_4$ Wasserstoff oder $C_1$—$C_6$-Alkyl ist.

5. Verbindung nach einem der Ansprüche 2, 3 oder 4, worin $R_6$ Wasserstoff darstellt und $R_7$ eine Gruppe der Formel $R_8CO$ ist, worin $R_8$ für Wasserstoff oder $C_1$—$C_6$-Alkyl steht.

6. Verbindung der Formel

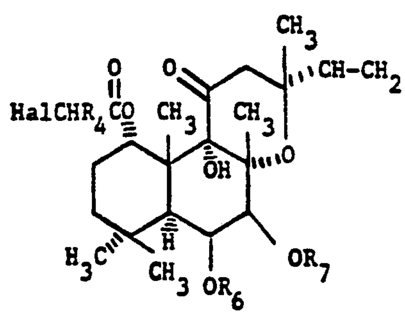

worin $R_4$ Wasserstoff, $C_1$—$C_6$-Alkyl oder Benzyl bedeutet; $R_6$ Wasserstoff oder eine Gruppe der Formel $R_5CO$ darstellt, worin $R_5$ Wasserstoff oder $C_1$—$C_6$-Alkyl ist; $R_7$ für Wasserstoff oder eine Gruppe der Formel $R_8CO$ steht, worin $R_8$ Wasserstoff oder $C_1$—$C_6$-Alkyl darstellt; Hal Chlor oder Brom bedeutet; oder die optischen und geometrischen Isomeren hievon.

7. Verbindung nach Anspruch 6, worin Hal Brom bedeutet.

8. Pharmazeutische Zusammensetzung, welche als wirksamen Bestandteil eine Verbindung nach Anspruch 1 in Verbindung mit einem pharmakologisch annehmbaren Träger umfaßt.

9. Verwendung einer Verbindung nach Anspruch 1 zur Herstellung eines Arzneimittels mit einer Wirksamkeit zur Verringerung von intraokularem Druck.

10. Verfahren zur Herstellung einer Verbindung der Formel

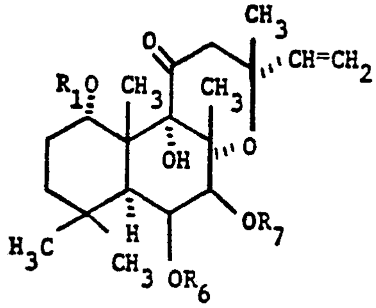

worin $R_1$ für $R_2R_3NCHR_4CO$ steht, worin $R_2$ Wasserstoff, $C_1$—$C_6$-Alkyl oder Benzyl bedeutet, $R_3$ Wasserstoff oder $C_1$—$C_6$-Alkyl bedeutet und $R_4$ Wasserstoff, $C_1$—$C_6$-Alkyl oder Benzyl ist, oder $R_2$ und $R_3$ zusammengenommen mit dem Stickstoffatom, an welches sie gebunden sind, eine Gruppe der Formel

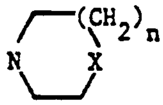

ausbilden, worin X CO, O, S, SO, $SO_2$, eine Gruppe der Formel $CHR_9$, worin $R_9$ Wasserstoff, $C_1$—$C_6$-Alkyl oder eine Gruppe der Formel $OR_{10}$ darstellt, worin $R_{10}$ für Wasserstoff oder $COR_{11}$ steht, worin $R_{11}$ $C_1$—$C_6$-Alkyl darstellt; oder eine Gruppe der Formel $NR_{12}$ bedeutet, worin $R_{12}$ $C_1$—$C_6$-Alkyl ist; n den Wert 1 oder 0 besitzt; $R_6$ für Wasserstoff oder eine Gruppe der Formel $R_5CO$ steht, worin $R_5$ Wasserstoff oder $C_1$—$C_6$-Alkyl bedeutet; $R_7$ Wasserstoff oder eine Gruppe der Formel $R_8CO$ darstellt, worin $R_8$ Wasserstoff oder $C_1$—$C_6$-Alkyl ist; oder $R_6$ und $R_7$ zusammengenommen eine Gruppe der Formel CO oder SO ausbilden; der optischen und geometrischen Isomeren hievon, oder eines pharmazeutisch annehmbaren Säureadditionssalzes hievon, welches:

(a) das Inkontaktbringen einer Verbindung der Formel

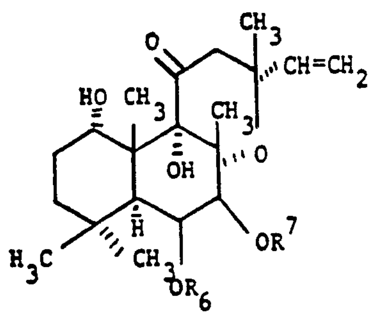

worin $R_6$ und $R_7$ wie oben beschrieben sind, mit einer Verbindung der Formel

$$HalCHR_4COHal,$$

worin $R_4$ wie oben beschrieben ist und Hal Brom oder Chlor darstellt, in Gegenwart eines tertiären Amins, um eine Verbindung der Formel

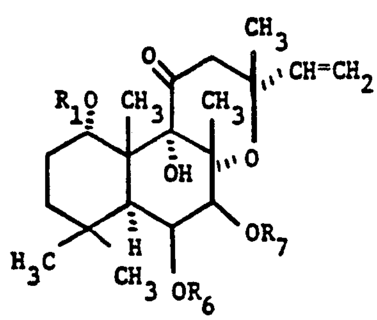

auszubilden, worin $R_1$ für $HalCHR_4CO$ steht, worin $R_4$ und Hal wie oben sind; und

(b) das Inkontaktbringen des Produktes aus Schritt (a) mit einer Verbindung der Formel $R_2R_3NH$, worin $R_2$ und $R_3$ wie oben sind, umfaßt.

**Patentansprüche für den Vertragstaat: AT**

1. Verfahren zur Herstellung einer Verbindung der Formel

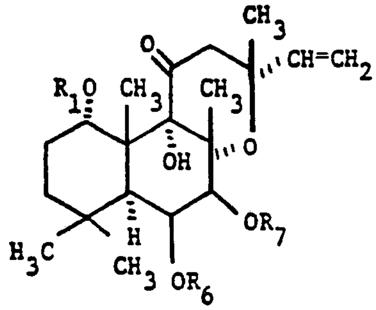

worin $R_1$ für $R_2R_3NCHR_4CO$ steht, worin $R_2$ Wasserstoff, $C_1$—$C_6$-Alkyl oder Benzyl bedeutet, $R_3$ Wasserstoff oder $C_1$—$C_6$-Alkyl bedeutet und $R_4$ Wasserstoff, $C_1$—$C_6$-Alkyl oder Benzyl ist, oder $R_2$ und $R_3$ zusammengenommen mit dem Stickstoffatom, an welches sie gebunden sind, eine Gruppe der Formel

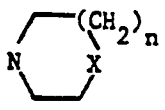

ausbilden, worin XCO, O, S, SO, $SO_2$, eine Gruppe der Formel $CHR_9$, worin $R_9$ Wasserstoff, $C_1$—$C_6$-Alkyl oder eine Gruppe der Formel $OR_{10}$ darstellt, worin $R_{10}$ für Wasserstoff oder $COR_{11}$ steht, worin $R_{11}$ $C_1$—$C_6$-Alkyl darstellt; oder eine Gruppe der Formel $NR_{12}$ bedeutet, worin $R_{12}$ $C_1$—$C_6$-Alkyl ist; n den Wert 1 oder 0 besitzt; $R_6$ für Wasserstoff oder eine Gruppe der Formel $R_5CO$ steht, worin $R_5$ Wasserstoff oder $C_1$—$C_6$-Alkyl bedeutet; $R_7$ Wasserstoff oder eine Gruppe der Formel $R_8CO$ darstellt, worin $R_8$ Wasserstoff oder $C_1$—$C_6$-Alkyl ist; oder $R_6$ und $R_7$ zusammengenommen eine Gruppe der Formel CO oder SO ausbilden; der optischen und geometrischen Isomeren hievon, oder eines pharmazeutisch annehmbaren Säureadditionssalz hievon, welches:

(a) das Inkontaktbringen einer Verbindung der Formel

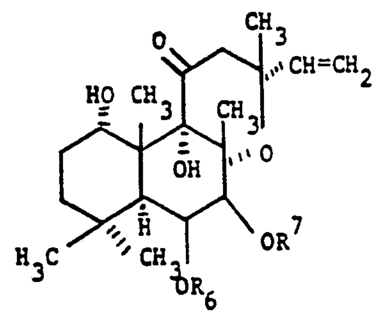

worin $R_6$ und $R_7$ wie oben beschrieben sind, mit einer Verbindung der Formel

$$HalCHR_4COHal,$$

worin $R_4$ wie oben beschrieben ist und Hal Brom oder Chlor darstellt, in Gegenwart eines tertiären Amins, um eine Verbindung der Formel

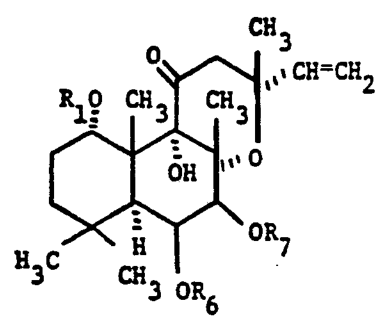

auszubilden, worin $R_1$ für $HalCHR_4CO$ steht, worin $R_4$ und Hal wie oben sind; und

(b) das Inkontaktbringen des Produktes aus Schritt (a) mit einer Verbindung der Formel $R_2R_3NH$, worin $R_2$ und $R_3$ wie oben sind, umfaßt.

2. Verfahren nach Anspruch 1, worin $R_2$ und $R_3$ beide $C_1$—$C_6$-Alkyl darstellen.

3. Verfahren nach Anspruch 1, worin $R_2$ und $R_3$ zusammengenommen mit dem Stickstoffatom, an welches sie gebunden sind, eine Grupe der Formel

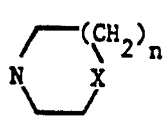

ausbilden, worin X für O, S oder eine Gruppe der Formel $CHR_9$ steht, worin $R_9$ Wasserstoff oder eine Gruppe der Formel $OR_{10}$ darstellt, worin $R_{10}$ Wasserstoff ist und n den Wert 0 der 1 bestizt.

4. Verfahren nach einem der Ansprüche 2 oder 3, worin $R_4$ Wasserstoff oder $C_1$—$C_6$-Alkyl ist.

5. Verfahren nach einem der Ansprüche 2, 3 oder 4, worin $R_6$ Wasserstoff darstellt und $R_7$ eine Gruppe der Formel $R_8CO$ ist, worin $R_8$ für Wasserstoff oder $C_1$—$C_6$-Alkyl steht.

6. Verfahren zur Herstellung einer Verbindung der Formel *2*

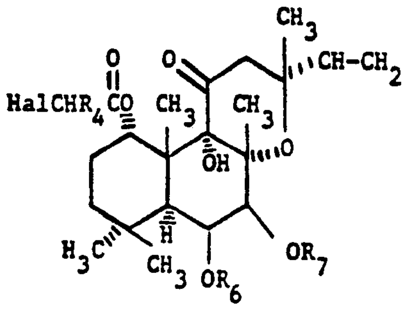

worin Hal, $R_4$, $R_6$ und $R_7$ wie in Anspruch 1 definiert sind, welches des Behandeln von einem Hydroxylabdan der Formel *3*

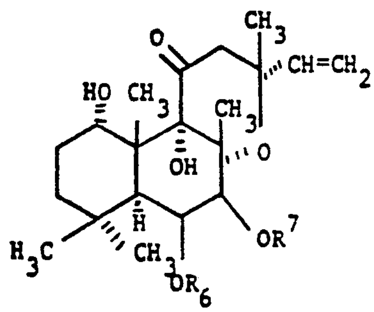

mit einem Halogen-niederalkylcarbonylhalogenid der Formel $HalCHR_4COHal$, worin Hal für Brom oder Chlor steht, in Gegenwart eines tertiären Amins umfaßt.

**Revendications pour les États contractants: BE CH DE FR GB IT LI LU NL SE**

1. Composé de formule

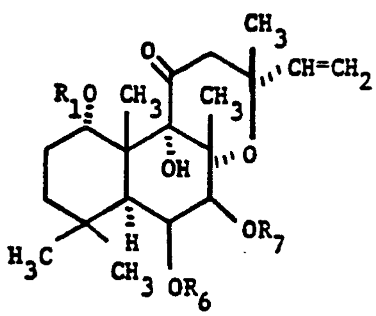

dans laquelle $R_1$ est $R_2R_3NCHR_4CO$ où $R_2$ est un atome d'hydrogène ou un groupe alkyle en $C_1$—$C_6$ ou benzyle, $R_3$ est un atome d'hydrogène ou un groupe alkyle en $C_1$—$C_6$ et $R_4$ est un atome d'hydrogène ou un groupe alkyle en $C_1$—$C_6$ ou benzyle, ou $R_2$ et $R_3$ ensemble, avec l'atome d'azote auquel ils sont fixés, forment un groupe de formule

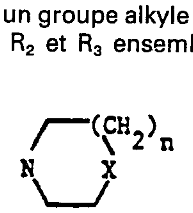

dans laquelle X est CO, O, S, SO ou $SO_2$; un groupe de formule $CHR_9$ dans laquelle $R_9$ est un atome d'hydrogène, un groupe alkyle en $C_1$—$C_6$ ou un groupe de formule $OR_{10}$ dans laquelle $R_{10}$ est un atome d'hydrogène ou $COR_{11}$ dans laquelle $R_{11}$ est un groupe alkyle en $C_1$—$C_6$; ou un groupe de formule $NR_{12}$ dans laquelle $R_{12}$ est un groupe alkyle en $C_1$—$C_6$; n vaut 1 ou 0; $R_6$ est un atome d'hydrogène ou un groupe de formule $R_5CO$ dans laquelle $R_5$ est un atome d'hydrogène ou un groupe alkyle en $C_1$—$C_6$; $R_7$ est un

atome d'hydrogène ou un groupe de formule $R_8CO$ dans laquelle $R_8$ est un atome d'hydrogène ou un groupe alkyle en $C_1$—$C_6$; ou $R_6$ et $R_7$ ensemble forment un groupe de formule CO ou SO; ses isomères optiques et géométriques, ou un sel d'addition de ce composé avec un acide pharmaceutiquement acceptable.

2. Composé selon la revendication 1, dans lequel $R_2$ et $R_3$ sont tous deux un groupe alkyle en $C_1$—$C_6$.

3. Composé selon la revendication 1, dans lequel $R_2$ et $R_3$ ensemble, avec l'atome d'azote auquel ils sont fixés, forment un groupe de formule

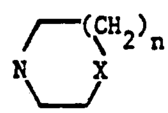

dans laquelle X est O, S ou un groupe de formule $CHR_9$ dans laquelle $R_9$ est un atome d'hydrogène, ou un groupe de formule $OR_{10}$ dans laquelle $R_{10}$ est un atome d'hydrogène, et n vaut 0 ou 1.

4. Composé selon l'une quelconque des revendications 2 ou 3, dans laquel $R_4$ est un atome d'hydrogène ou un groupe alkyle en $C_1$—$C_6$.

5. Composé selon l'une quelconque des revendications 2, 3 ou 4, dans lequel $R_6$ est un atome d'hydrogène et $R_7$ est un groupe de formule $R_8CO$ dans laquelle $R_8$ est un atome d'hydrogène ou un groupe alkyle en $C_1$—$C_6$.

6. Composé de formule

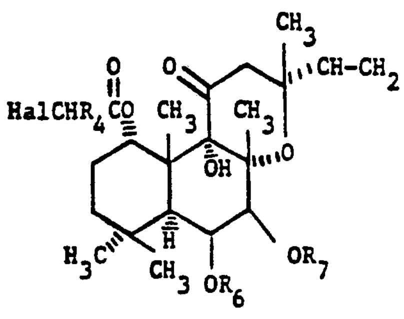

dans laquelle $R_4$ est un atome d'hydrogène ou un groupe alkyle en $C_1$—$C_6$ ou benzyle; $R_6$ est un atome d'hydrogène ou un groupe de formule $R_5CO$ dans laquelle $R_5$ est un atome d'hydrogène ou un groupe alkyle en $C_1$—$C_6$; $R_7$ est un atome d'hydrogène ou un groupe de formule $R_8CO$ dans laquelle $R_8$ est un atome d'hydrogène ou un groupe alkyle en $C_1$—$C_6$; Hal est un groupe chloro ou bromo; ou ses isomères optiques et géométriques.

7. Composé selon la revendication 6, dans lequel Hal est un groupe bromo.

8. Composition pharmaceutique qui comprend comme ingrédient actif un composé selon la revendication 1 en association avec un véhicule pharmacologiquement acceptable.

9. Utilisation d'un composé selon la revendication 1 pour la préparation d'un médicament ayant une activité d'abaissement de la pression intra-oculaire.

10. Procédé pour la préparation d'un composé de formule

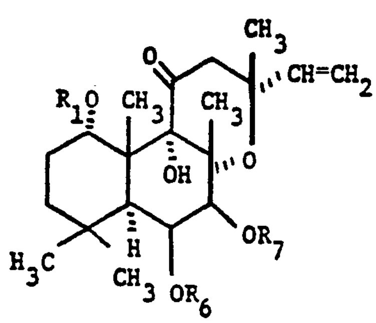

dans laquelle $R_1$ est $R_2R_3NCHR_4CO$ où $R_2$ est un atome d'hydrogène ou un groupe alkyle en $C_1$—$C_6$ ou benzyle, $R_3$ est un atome d'hydrogène ou un groupe alkyle en $C_1$—$C_6$ et $R_4$ est un atome d'hydrogène ou un groupe alkyle en $C_1$—$C_6$ ou benzyle; ou $R_2$ et $R_3$ ensemble, avec l'atome d'azote auquel ils sont fixés, forment un groupe de formule

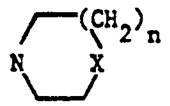

dans laquelle X est CO, O, S, SO ou $SO_2$; un groupe de formule $CHR_9$ dans laquelle $R_9$ est un atome d'hydrogène, un groupe alkyle en $C_1$—$C_6$ ou un groupe de formule $OR_{10}$ dans laquelle $R_{10}$ est un atome d'hydrogène ou $COR_{11}$ dans laquelle $R_{11}$ est un groupe alkyle en $C_1$—$C_6$; ou un groupe de formule $NR_{12}$ dans laquelle $R_{12}$ est un groupe alkyle en $C_1$—$C_6$; n vaut 1 ou 0; $R_6$ est un atome d'hydrogène ou un groupe de formule $R_5CO$ dans laquelle $R_5$ est un atome d'hydrogène ou un groupe alkyle en $C_1$—$C_6$; $R_7$ est un atome d'hydrogène ou un groupe de formule $R_8CO$ dans laquelle $R_8$ est un atome d'hydrogène ou un groupe alkyle en $C_1$—$C_6$; ou $R_6$ et $R_7$ ensemble forment un groupe de formule CO ou SO; ses isomères optiques et géométriques, ou un sel d'addition de ce composé avec un acid pharmaceutiquement acceptable, qui comprend:

(a) le contact d'un composé de formule

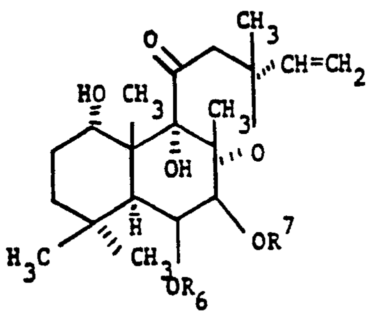

dans laquelle $R_6$ et $R_7$ sont comme décrit ci-dessus, avec un composé de formule

$HalCHR_4COHal$

dans laquelle $R_4$ est comme décrit ci-dessus et Hal est un groupe bromo ou chloro, en présence d'une amine tertiaire pour fournir un composé de formule

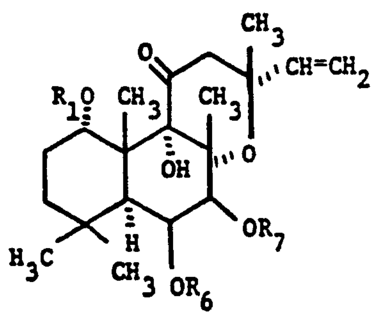

dans laquelle $R_1$ est $HalCHR_4CO$ où $R_4$ et Hal sont comme ci-dessus; et

(b) le contact du produit de l'étape (a) avec un composé de formule $R_2R_3NH$ dans laquelle $R_2$ et $R_3$ sont comme ci-dessus.

**Revendications pour Etat contractant: AT**

1. Procédé pour la préparation d'un composé de formule

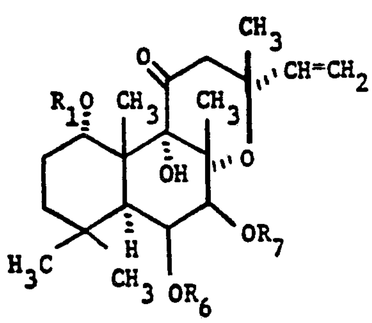

dans laquelle $R_1$ est $R_2R_3NCHR_4CO$ où $R_2$ est un atome d'hydrogène ou un groupe alkyle en $C_1$—$C_6$ ou benzyle, $R_3$ est un atome d'hydrogène ou un groupe alkyle en $C_1$—$C_6$ et $R_4$ est un atome d'hydrogène ou un groupe alkyle en $C_1$—$C_6$ ou benzyle, ou $R_2$ et $R_3$ ensemble, avec l'atome d'azote auquel ils sont fixés, forment un groupe de formule

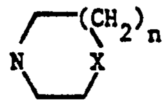

dans laquelle X est CO, O, S, SO ou $SO_2$; un groupe de formule $CHR_9$ dans laquelle $R_9$ est un atome d'hydrogène, un groupe alkyle en $C_1$—$C_6$ ou un groupe de formule $OR_{10}$ dans laquelle $R_{10}$ est un atome d'hydrogène ou $COR_{11}$ dans laquelle $R_{11}$ est un groupe alkyle en $C_1$—$C_6$; ou un groupe de formule $NR_{12}$ dans laquelle $R_{12}$ est un groupe alkyle en $C_1$—$C_6$; n vaut 1 ou 0; $R_6$ est un atome d'hydrogène ou un groupe de formule $R_5CO$ dans laquelle $R_5$ est un atome d'hydrogène ou un groupe alkyle en $C_1$—$C_6$; $R_7$ est un atome d'hydrogène ou un groupe de formule $R_8CO$ dans laquelle $R_8$ est un atome d'hydrogène ou un groupe alkyle en $C_1$—$C_6$; ou $R_6$ et $R_7$ ensemble forment un groupe de formule CO ou SO; ses isomères optiques et géométriques, ou un sel d'addition de ce composé avec un acide pharmaceutiquement acceptable, qui comprend:

(a) le contact d'un composé de formule

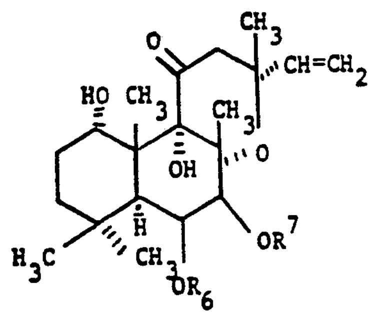

dans laquelle $R_6$ et $R_7$ sont comme décrit ci-dessus, avec un composé de formule

$$HalCHR_4COHal$$

dans laquelle $R_4$ est comme décrit ci-dessus et Hal est un groupe bromo ou chloro, en présence d'une amine tertiaire pour fournir un composé de formule

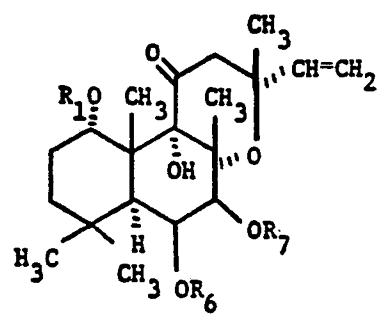

dans laquelle $R_1$ est $HalCHR_4CO$ où $R_4$ et Hal sont comme ci-dessus; et

(b) le contact du produit de l'étape (a) avec un composé de formule $R_2R_3NH$ dans laquelle $R_2$ et $R_3$ sont comme ci-dessus.

2. Procédé selon la revendication 1, dans lequel $R_2$ et $R_3$ sont tous deux un groupe alkyle en $C_1$—$C_6$.

3. Procédé selon la revendication 1, dans lequel $R_2$ et $R_3$ ensemble, avec l'atome d'azote auquel ils sont fixés, forment un groupe de formule

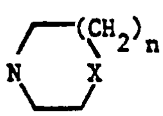

dans laquelle X est O, S ou un groupe de formule $CHR_9$ dans laquelle $R_9$ est un atome d'hydrogène, ou un groupe de formule $OR_{10}$ dans laquelle $R_{10}$ est un atome d'hydrogène, et n vaut 0 ou 1.

4. Procéde selon l'une quelconque des revendications 2 ou 3, dans laquel $R_4$ est un atome d'hydrogène ou un groupe alkyle en $C_1$—$C_6$.

5. Procédé selon l'une quelconque des revendications 2, 3 ou 4, dans lequel $R_6$ est un atome d'hydrogène et $R_7$ est un groupe de formule $R_8CO$ dans laquelle $R_8$ est un atome d'hydrogène ou un groupe alkyle en $C_1$—$C_6$.

6. Procédé pour la préparation d'un composé de formule *2*

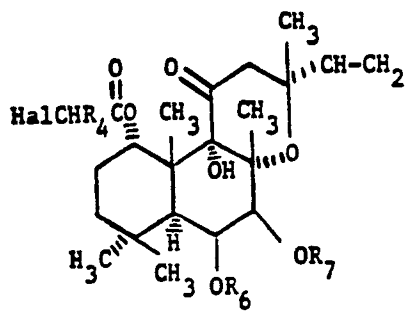

dans laquelle Hal, $R_4$, $R_6$ et $R_7$ sont comme défini dans la revendication 1, qui comprend le traitement d'un hydroxylabdane de formule *3*

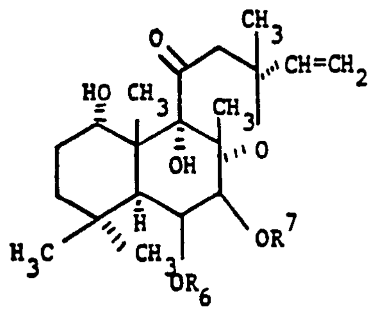

avec un halogénure d'halogéno(alkyl inférieur)carbonyle de formule $HalCHR_4COHal$ dans laquelle Hal est un groupe bromo ou chloro, en présence d'une amine tertiaire.